(19) Europäisches Patentamt
European Patent Office
Office européen des brevets

(11) Veröffentlichungsnummer: **0 595 228 A2**

(12) **EUROPÄISCHE PATENTANMELDUNG**

(21) Anmeldenummer: **93117235.7**

(22) Anmeldetag: **25.10.93**

(51) Int. Cl.5: **C07C 43/305**, C07C 43/303, C07C 41/50

(30) Priorität: **28.10.92 DE 4236321**
**28.08.93 DE 4329033**

(43) Veröffentlichungstag der Anmeldung:
**04.05.94 Patentblatt 94/18**

(84) Benannte Vertragsstaaten:
**DE GB IT NL**

(71) Anmelder: **HÜLS AKTIENGESELLSCHAFT**

**D-45764 Marl(DE)**

(72) Erfinder: **Steffen, Klaus-Dieter Dr.**
**Röckelstrasse 36**
**D-53773 Hennef(DE)**
Erfinder: **Metz, Josef Dr.**
**Am Erzschacht 31**
**D-45770 Marl(DE)**

(54) **Verfahren zur Herstellung von Ketalen.**

(57) Ketale von längerkettigen bzw. verzweigten Alkoholen werden aus den Dimethylketalen bzw. den Ketonen durch Reaktion der Dimethylketale und den Alkoholen bei hohen Temperaturen von 105 bis 180 °C in Gegenwart von sauren Katalysatoren erhalten. Die durch Destillation abgetrennten Enolether und Mischketale werden einem nächsten Ansatz zugesetzt und reagieren ebenfalls zum Zielprodukt.
Der Ausgangsstoff Dimethylketal kann auch während der Reaktion aus dem Keton und Trimethlorthoformiat gebildet werden.

EP 0 595 228 A2

Die Erfindung betrifft ein verfahren zur Herstellung von Ketalen durch sauer katalysierte Umketalisierung von Dimethylketalen mit mindestens einer längerkettigen Alkylgruppe, einem Arylrest und besonders von Cyclohexanon mit längerkettigen bzw. sec. Alkoholen.

Die Herstellung von Ketalen ist nur aus einfachen Ketonen, besonders aus Aceton, und kurzkettigen Alkoholen durch Synthese aus den Bestandteilen oder Umketalisierung ohne Schwierigkeiten ausführbar. Es ergeben sich jedoch stets wechselnde Anteile von Enol-Alkylethern, je nach Struktur der Bestandteile und den Reaktionsbedingungen.

Zur Erhöhung des Umsatzgrades werden saure Katalysatoren, Molekularsiebe bzw. Ionenaustauscher, trotz deren schwieriger Handhabbarkeit, eingesetzt.

Bessere Umsätze und Ausbeuten an Ketalen werden durch Umsetzung von Ketonen mit Orthoameisensäureestern erzielt, von denen aber nur die Methyl- und Ethyl-orthoester befriedigend herstellbar sind. Ketale der nicht einfachen Struktur der nachstehenden Formel II sind auch durch Umketalisierung aus den Dimethyl-Ketalen, beispielsweise nach US-PS 3,072,727, herstellbar, zu deren Herstellung 2,2-Di-Methoxipropan (DMP aus Aceton) dient. Ketale der Formel II, z.B. Cyclohexanon-di-isopropylketal, werden nur in Ausbeuten von 30% (US-PS 3,072,727 Beispiel VI) neben großen Mengen verschiedener unsymmetrischer Ketale erhalten. DMP erfordert die Verwendung von Schleppmitteln wie Hexan oder Benzol, die mit DMP aber ein Azeotrop bilden. In der US-PS 3,072,727 (Patentanspruch Zeile 40) wird ausdrücklich festgehalten, daß die Reaktionstemperatur 100 °C nicht überschreiten darf, da sonst große Mengen verschiedener Cyclohexen-alkylether entstehen.

Gegenstand der Erfindung ist daher ein Verfahren zur Herstellung von Ketalen durch Umketalisierung von Dimethylketalen mit Alkoholen der Formel $R^2OH$ in Gegenwart von sauren Katalysatoren und Abdestillation des Methanols, dadurch gekennzeichnet, daß nach der allgemeinen Formel

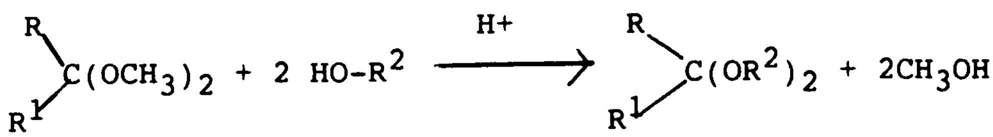


I III II

$R,R^1$ = Alkylreste mit 1 bis 8 C-Atomen oder Arylreste
oder
$R + R^1$ = $-(CH_2)_5$ - oder - $(CH_2)_4$ -
$R^2$ = lineare oder verzweigte Alkylreste mit 2 bis 8 C-Atomen

das Dimethylketal mit den Alkoholen $R^2OH$ zu mehr als 90% bei Temperaturen von 105 bis 180 °C umgesetzt und die Ketale der Alkohole $R^2OH$ durch Destillation gewonnen werden und die Enol-alkylether in die Reaktion zurückgeführt werden.

Die Alkohole $R^2OH$ haben vorzugsweise Alkylreste von 3 bis 8 C-Atomen, sehr bevorzugt verzweigte Alkylreste.

Ausgangsstoff ist das Dimethyl-Ketal des jeweiligen Ketons in vorgebildeter Form oder durch vorangehende Herstellung aus dem Keton und Trimethylorthoformiat (TMOF). Diese bekannte Reaktion verläuft exotherm bei 20 bis 80 °C mit nahezu quantitativer Ausbeute unter Zusatz saurer Katalysatoren.

Das erfindungsgemäße Verfahren ist besonders zur Herstellung von Ketalen der Formel II geeignet, worin im Keton oder im Rest $R_2$ mindestens eine Alkylgruppe mit 3 bis 8 Kohlenstoffatomen oder ein Arlyrest bzw. Cycloalkylrest mit einem Ring enthalten ist und/oder worin die Alkylreste der Alkoholkomponente längerkettige lineare Alkohole mit 2 bis 8 Kohlenstoffatomen, besonders verzweigte Alkylreste, sind. Ganz besonders ist jedoch das Verfahren für die Herstellung von Cyclohexyl- oder Cyclopentylketalen der verzweigten Alkohole, insbesondere von Isopropanol und Isobutanol, geeignet. Das vorliegende Verfahren wird bewußt bei hohen Temperaturen von 105 bis 180 °C, vorzugsweise 110 bis 180 °C und sehr bevorzugt 120 bis 180 °C, durchgeführt. Dabei wird durch Reaktion aller Methoxigruppen und durch Abdestillation als Methanol ein Umsatz zu Acetalen der Alkohole der Formel III mit dem Keton von 90% oder mehr ausgeführt. Von besonderem Vorteil sind Endtemperaturen von 150 bis 180 °C bei der Herstellung der Produkte der Formel II.

Die Bildung von Enol-Alkylethern, d.h. Ethern der ungesättigten Alkohole, die dem Alkanol $R^2OH$ entsprechen, wird bewußt in Kauf genommen.

Es wurde nämlich gefunden, daß das Zielprodukt der Formel II den höchsten Siedepunkt von allen Reaktionsprodukten besitzt, der im Durchschnitt 20 °C oberhalb der übrigen Ketale und der Ether liegt. Der

Enol-ether fällt in Mengen von 0 bis 30 Mol-% an, stellt jedoch keinen Verlust dar, da er ebenso wie die Mischketale, als ggf. gemeinsame Fraktion, durch Destillation abgetrennt und ungereinigt einem nachfolgenden Ansatz oder demselben Ansatz zugefügt werden kann. Vor dieser Destillation werden alle Leichtsieder, nämlich Methanol und der Überschuß der Alkohole neben Estern und Ethern, abgetrennt.
Durch die hohe Temperatur kann die Reaktion in kurzer Zeit ausgeführt werden. Es besteht der erhebliche Vorteil, daß Schleppmittel, im Gegensatz zum Stand der Technik, nicht verwendet werden.
Bei der Reaktion sind als Katalysatoren saure Verbindungen zu verwenden, obgleich in bekannter Weise Acetale und Ketale unter sauren Bedingungen Alkohol abspalten.

Als saure Katalysatoren kommen z.B. p-Toluol-sulfonsäure, Schwefelsäure, saure Ionenaustauscher, Bortrifluoridkomplexe, Ammonchlorid u.a. in Mengen von 0,1 bis 0,4 g Säure pro Mol Keton infrage.
Die sauren Katalysatoren sind zweckmäßig bereits anwesend, soweit das Dimethylketal vorangehend aus TMOF hergestellt wird.
Die sauren Katalysatoren sind vor der Destillation durch Zusatz von Alkalien, zweckmäßg durch Zusatz von Alkalialkoholaten, besonders Na-Alkoholaten, besonders des Alkohols der Formel III, in mindestens äquivalenter Menge zu neutralisieren.

Weiterer Gegenstand der Erfindung ist ein Verfahren zur Herstellung von Ketalen der Formel II, bei dem gleichzeitig mit der Herstellung dieser Ketale aus Dimethylketalen und dem Alkohol der Formel III das Dimethylketal der Formel I aus dem jeweiligen Keton und Trimethylorthoformiat gebildet wird.
Zweckmäßig sind hierbei die genannten sauren Katalysatoren bereits anwesend. Es kann bei dieser Verfahrensweise ein Teil des Alkohols der Formel III vorgelegt werden und ein weiterer Teil später zugefügt werden.
Dabei werden vorteilhaft das Keton, z.B. Cyclohexanon, und Isopropanol vorgelegt, Trimethylorthoformiat bei möglichst hoher Sumpftemperatur zugefügt und Methylformiat und Methanol abdestilliert.
Geeignete Temperaturen am Beginn der Reaktion liegen zwischen 75 und 120 °C. Durch Zugabe von Isopropanol werden Mischketale in Diisopropylketal überführt.
Ausbeuten von 90 bis 96% reiner Ketale, bezogen auf das Keton, sind erreichbar.
Letztlich werden gemäß der Erfindung die im Stand der Technik in großen Mengen entstehenden Mischketale und Enolether fast gänzlich vermieden.

**Beispiel 1: Cyclohexanon-di-isopropylketal (CHiPK)**

In einem 1 l-Vierhalskolben, versehen mit Rührer, Thermometer und Kolonne zur fraktionierten Destillation, werden 196,3 g Cyclohexanon (2,0 Mol), 10,9 g Methanol (0,34 Mol) und 0,4 g p-Toluolsulfonsäure vorgelegt. Bei Kühlung im Wasserbad werden 212,5 g Trimethylorthoformiat (TMOF, 2,0 Mol) in 1 Std. bei 18 bis 25 °C zugetropft und der Umsatz durch Nachreaktion (1,5 Std.) vervollständigt.

Nach G.C. beträgt die Zusammensetzung:
85% Cyclohexanon-di-methylketal (CHMK)
2% Methanol
12% Methylformiat
und Spuren an Cyclohexanon.

Zu dieser Mischung von Produkten werden in dem gleichen Kolben 481 g Isopropanol (8 Mol) zugesetzt und die Reaktionsmischung aufgeheizt, wobei unter Rückfluß alle Leichtsieder, d.h. Ameisensäuremethylester, Methanol, Ameisensäureisopropylester und Isopropanol in 2 St. abdestillieren, wobei die Reaktionstemperatur allmählich auf 125 bis 130 °C gesteigert wird. Nach der Hälfte der Zeit werden nochmals 0,4 g p-Toluolsulfonsäure zugesetzt.

Nach 15 Std. ist der Umsatz auf 95% gebracht und weist im Mittel folgende Zusammensetzung auf:
45% CHiPK
40% Isopropanol
6% Cyclohexen-isopropylether
5% Cyclohexanon-methyl-isopropyl-mischketal.

Durch Zugabe von 1 g Natrium-isopropylat wird die p-Toluolsulfonsäure neutralisiert. Bei partiellem Vakuum von 500 bis 250 hPa wird reines Isopropanol abdestilliert, das in die Folgeansätze rückgeführt wird. Nach Verbesserung des Vakuums auf 20 hPa wird eine zweite Fraktion unter Rückfluß abdestilliert, die den Cyclohexen-isopropylether, die Mischketale, geringe Anteile an Isopropanol und Cyclohexanon und Anteile von CHiPK enthält. Danach wird bei einer Kopftemperatur von 89 bis 90 °C/20 hPa (65 °C/5 hPa) das Cyclohexanon-di-isopropylketal als Zielprodukt abdestilliert.
Ausbeute: 250 g (62,4% d.Th., bezogen auf eingesetztes Cyclohexanon)
G.C.-Reinheit: 98,3 % CHiPK

**Beispiele 2 bis 5: Cyclohexanon-di-isoproylketal (CHiPK)**

Wie in Beispiel 1 beschrieben, wurde der Ausgangsstoff Cyclohexanon-di-methylketal hergestellt. Das Ketal wurde wie in Beispiel 1 hergestellt, jedoch die zweite Fraktion aus vorangehenden Ansätzen mit besonderen Gehalten von Cyclohexen-isopropylether im Mittel zugesetzt und entsprechend frisches Cyclohexanon bzw. dessen Dimethylketal auf 2,0 Mol ergänzt.

Das Isopropanol bestand zum Teil aus der Rückgewinnung.

Die Durchführung der Umketalisierung und der Auf- sowie Reindestillation erfolgte wie in Bespiel 1 beschrieben.

Ausbeuten und Angaben zu den Beispielen 2 bis 5 sind in der nachfolgenden Tabelle aufgeführt.

| Reaktionsparameter | Beispiele 2 | 3 | 4 | 5 |
|---|---|---|---|---|
| **Einsatzstoffe:** | | | | |
| CHMK-roh (g/Mol) | 285/1,34 | 292/1,39 | 274/1,31 | 279/1,33 |
| Isopropanol | | | | |
| frisch (g/Mol) | 333/5,55 | 278/4,63 | 253/4,22 | 237/3,94 |
| Rückgew. (g/Mol) | 148/2,45 | 203/3,37 | 227/3,78 | 244/4,06 |
| Vorlauffraktion von vorher. Ansatz | 1 | 2 | 3 | 4 |
| (g/Mol) | 113/0,66 | 96/0,61 | 104/0,69 | 114/0,67 |
| p-Toluolsulfonsäure (g) | 0,4 | 0,4 | 0,4 | 0,4 |
| Na-Isopropylat (g) | 1 | 1 | 1 | 1 |
| **CHiPK-Reindestillat:** | | | | |
| Menge (g) | 258 | 248 | 237 | 245 |
| Reinheit (GC-%) | 98.0 | 98,3 | 98,8 | 98,3 |
| Ausb. (% d.Th.) (bezogen auf CHMK) | 96,1 | 89.0 | 90,3 | 91,9 |

CHMK = Cyclohexanon-dimethylketal

**Beispiel 6: Cyclohexanon-di-isobutylketal (CHiBK)**

Wie in Beispiel 1 beschrieben, wurde Cylcohexanon-dimethylketal (CHMK) aus 1 Mol Cyclohexanon und Trimethyl-orthoformiat hergestellt.

Nach Zugabe von 370,6 g Isobutanol (2-Methyl-propanol-1, 5,0 Mol) wurde bei allmählich von 110° auf 160°C ansteigender Sumpftemperatur unter Rückfluß die Umketalisierung durchgeführt. Nach 2 Std. Laufzeit wurden nochmals 0,4 g p-Toluolsulfonsäure zugegeben.

Die Leichtsieder, d.h. Methylformiat, Methanol mit etwas Isobutylformiat, wurden unter Rückfluß (Verhältnis

1:5 bis 1:2) in 4 Std. abdestilliert.

Nachdem die Sumpfanalyse Werte von 79% CHiBK, 16% Isobutanol, je 1% Methylisobutyl-Mischketal, Cyclohexanon und Cyclohexen-ether aufwies (über 90% Umsatz von Cyclohexanon), wurde mit 1 g Na-methylat neutralisiert und die Destillation begonnen. Bei vermindertem Druck von zunächst 20 hPa, dann verbessert auf 5 hPa, wurden Isobutanol und die anderen Nebenprodukte abdestilliert. Bei einem Siedepunkt von 98°C/5 hPa ohne Rückfluß wurde das Cyclohexanon-di-isobutylketal über Kopf abgenommen. Als Vorlauf wurden vorher 95 g abdestilliert (65% Isobutanol, 17% CHiBK, Cyclohexanon, Cyclohexenether sowie Mischketalen), zusammen 0,21 Mol CHiBK, Cyclohexanon und dessen Derivate.

Der Vorlauf wurde in Beispiel 7 wieder eingesetzt.

Ausbeute: 352 g (75,5% d.Th., bezogen auf Cyclohexanon))
Reinheit: 98,0 % (G.C.)

**Beispiel 7: Cyclohexanon-di-isobutylketal (CHiBK)**

CHMK wurde in einem 2 Mol-Ansatz wie in Beispiel 6 hergestellt und nach Zugabe von 370,6 g Isobutanol (5,0 Mol) sowie 95 g der Vorlauf-Fraktion aus Beispiel 6 bei von 105°C auf 160°C ansteigender Sumpftemperatur umketalisiert.

Das gesamte Methanol, der Ameisensäureester und etwas Isobutanol destillierten bei einem Rückfluß von 1:2 im Laufe von 4 Std. ab. Die Reaktionslösung wies dann eine Zusammensetzung von 23% Isobutanol, 68% CHiBK, je 2% Cyclohexanon und Mischketal neben wenig Cyclohexenether auf. Mit 0,5 g Na-methylat wurde der saure Katalysator neutralisiert und die Mischung bei allmählich von 20 hPa auf 5 hPa besser werdendem Vakuum aufdestilliert. Als Vorlauf destillierten 177 g ab, die zu 75% aus Isobutanol, 10% aus CHiBK sowie restlichem Cyclohexanon, Cyclohexenether und Mischketal mit zusammen 0,251 Mol an potentiellem CHiBK (12,6% Ausbeute) bestanden. Das Zielpro dukt destillierte bei 97 bis 98°C/5 hPa über.

CHiBK-Reinheit: 98,4% (G.C.)
Ausbeute: 394 g = 84,9% d.Th., bezogen auf 2,0 Mol Cyclohexanon, 88,2% d.Th., einschließlich der Vorläufe

**Beispiel 8: Herstellung CHiPK**

**Einsatzmengen:**

327,2 g = 3,33 Mol Cyclohexanon
0,67 g = 0,004 Mol pTSs
400,0 g = 6,66 Mol Isopropanol (vorlegen)
392,5 g = 6,53 Mol Isopropanol (zudosieren)
353,7 g = 3,33 Mol TMOF

**Apparatur:**

Beheizter 2 l-Rührkolben, Tropftrichter, 1m-Kolonne mit Multifl-Füllkörper, Dampfteiler, Rückflußkühler und Destillatvorlage-System unter $N_2$-Abdeckung.

**Durchführung:**

Cyclohexanon, Isopropanol und pTSs werden vorgelegt. Die Temperatur wird auf 80°C eingestellt und TMOF innerhalb von 3 h zugetropft. Das dabei entstandene Methylformiat/Methanol-Gemisch wird über Kopf abgezogen (Kopftemperatur 40 bis 60°C).

Anschließend wird die Sumpftemperatur stufenweise bis 115°C angehoben und mit hohem Rücklauf weiter Methanol über Kopf abgezogen. Nach einer Laufzeit von 10 h wird Isopropanol zudosiert.

Nach einer Laufzeit von 30 h beträgt der Anteil von CHiPK (in FID-Flächen-% ) 56.7.

**Aufarbeitung:**

Die Reaktionsmischung wird mit Na-methylat in methanolischer Lösung versetzt. Bei 300 hPa und 40°C Kopftemperatur wird bei einem Rücklaufverhältnis von 1:1 eine Fraktion aus Isopropanol und niedrig siedenden Nebenprodukten abgetrennt, die einem Folgeansatz zugesetzt wird.

Darauf erfolgt die Reindestillation bis 5 hPa und 85°C. Das Produkt hat einen Gehalt von über 97,5 %

CHiPK (FID-Flächen-%).

**Patentansprüche**

**1.** Verfahren zur Herstellung von Ketalen durch Umketalisierung von Dimethylketalen mit Alkoholen der Formel $R^2OH$ in Gegenwart von sauren Katalysatoren und Abdestillation des Methanols, **dadurch gekennzeichnet,** daß nach der allgemeinen Formel

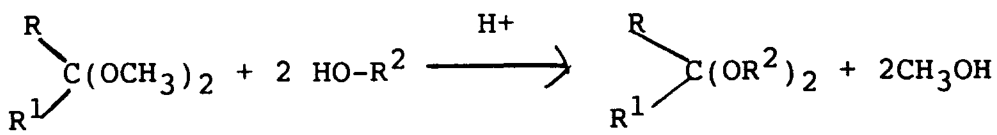

I III II

R,$R^1$ = Alkylreste mit 1 bis 8 C-Atomen oder Arylreste
oder
R + $R^1$ = $-(CH_2)_5$ - oder - $(CH_2)_4$ -
$R^2$ = lineare oder verzweigte Alkylreste mit 2 bis 8 C-Atomen
das Dimethylketal mit den Alkoholen $R^2OH$ zu mehr als 90% bei 105 bis 180 °C umgesetzt und die Ketale der Alkohole $R^2OH$ durch Destillation gewonnen werden und die Enol-alkylether in die Reaktion zurückgeführt werden.

**2.** Verfahren nach Anspruch 1, **dadurch gekennzeichnet,** daß vor der Destillation die sauren Katalysatoren neutralisiert werden.

**3.** Verfahren nach Anspruch 2, **dadurch gekennzeichnet,** daß die Enol-alkylether einem nachfolgenden Ansatz zugegeben werden.

**4.** Verfahren nach einem der vorangehenden Ansprüche, **dadurch gekennzeichnet,** daß Ketale des Cyclohexanons bzw. Cyclopentanons hergestellt werden.

**5.** Verfahren nach einem der vorangehenden Ansprüche, **dadurch gekennzeichnet,** daß die Umsetzung bei 120 bis 180 ° C erfolgt.

**6.** Verfahren nach einem der vorangehenden Ansprüche, **dadurch gekennzeichnet,** daß die Endtemperatur der Umsetzung 150 bis 180 °C beträgt.

**7.** Verfahren nach Anspruch 1, **dadurch gekennzeichnet,** daß zunächst das Dimethylketal der Formel I durch Reaktion von Trimethylorthoformiat mit dem zugehörigen Keton in Gegenwart von sauren Katalysatoren gebildet und der Ansatz ohne Aufarbeitung zu den Ketalen II umgesetzt wird.

**8.** Verfahren zur Herstellung von Ketalen nach Anspruch 1, **dadurch gekennzeichnet,** daß gleichzeitig mit der Herstellung von Ketalen aus Dimethylketalen das Dimethylketal der Formel I aus dem jeweiligen Keton und Trimethylorthoformiat gebildet wird.

**9.** Verfahren nach Anspruch 8, **dadurch gekennzeichnet,** daß ein saurer Katalysator anwesend ist.

**10.** Verfahren nach Anspruch 8 oder 9, **dadurch gekennzeichnet,** daß am Beginn der Reaktion die Temperatur zwischen 75 und 120 °C liegt.

**11.** Verfahren nach einem der Ansprüche 1 bis 10, **dadurch gekennzeichnet,** daß die bei der Gewinnung von Ketalen der Formel II abgetrennten Nebenprodukte, besonders Mischketale und Ausgangsstoffe, der Reaktionsmischung zugegeben werden.